# EUROPEAN PATENT APPLICATION

(11) **EP 4 435 012 A1**
(43) Date of publication of application: **25.09.2024**
(21) Application number: 22894890.7
(22) Date of filing: 17.11.2022
(51) Int. Cl.: C07K 19/00, C12N 15/62, C12N 15/867, C12N 5/10, A61K 39/00, A61P 35/00

(54) **BCMA-TARGETING CHIMERIC ANTIGEN RECEPTOR AND USE THEREOF**

(30) Priority: 18.11.2021 CN 202111374219
(71) Applicant: Juventus Cell Therapy Ltd., Tianjin 300384 (CN)
(72) Inventor: BAI, Dayong, Tianjin 300384 (CN); ZHANG, Yunlong, Tianjin 300384 (CN); ZHANG, Chao, Tianjin 300384 (CN); LV, Lulu, Tianjin 300384 (CN); ZHOU, Li, Tianjin 300384 (CN); WANG, Yongzeng, Tianjin 300384 (CN); WANG, Rui, Tianjin 300384 (CN); DING, Wei, Tianjin 300384 (CN); LU, Jiaxing, Tianjin 300384 (CN); ZHANG, Qimeng, Tianjin 300384 (CN)
(74) Representative: Ponti & Partners, S.L.P
(86) International application number: PCT/CN2022/132545
(87) International publication number: WO 2023/088359

(57) **Abstract**

Provided in the present invetion is a BCMA-targeting chimeric antigen receptor, comprising an extracellular antigen recognition domain, a hinge region, a transmembrane region and an intracellular domain. The extracellular antigen recognition domain comprises an anti-BCMA scFv antibody. The amino acid sequences of VH complementarity determining regions CDR1, CDR2, and CDR3 of the scFv antibody respectively comprise amino acid sequences as shown in SEQ ID NO: 1, SEQ ID NO: 2, and SEQ ID NO: 3. The amino acid sequences of VL complementarity determining regions CDR1, CDR2, and CDR3 of the scFv antibody respecitvley comprise amino acid sequences as shown in SEQ ID NO: 4, SEQ ID NO: 5, and SEQ ID NO: 6.

## Description

### TECHNICAL FIELD

The present application relates to the field of biomedicine, specifically to BCMA-targeting chimeric antigen receptor and use thereof.

### BACKGROUND

Multiple myeloma, defined as a malignant proliferation of plasma cells in the bone marrow, is the second most common hematological malignancy, accounting for 1% of all cancer types. Studies have shown that multiple myeloma is more common among people over 60 years old and its incidence rate has increased steadily in recent years. For most patients, multiple myeloma is incurable and eventually develops into relapsed/refractory multiple myeloma. The survival time of patients with relapsed/refractory multiple myeloma who are ineffective against existing multiple myeloma treatments (such as immunomodulators, proteasome inhibitors, and antibody drugs) is only about 13 months.

B Cell Maturation Antigen (BCMA) is a transmembrane glycoprotein that belongs to the tumor necrosis factor receptor family. BCMA is highly expressed in multiple myeloma cells and not in most other cells. Malignant tumor plasma cells usually express higher levels of BCMA than those of normal plasma cells, the upregulation of BCMA promotes the growth of multiple myeloma cancer cells, while the downregulation of its expression can inhibit the growth of multiple myeloma cancer cells.

Chimeric Antigen Receptor (CAR) is the core component of CAR cell therapy drugs, which can include a targeting portion (for example, a portion that binds to Tumor-Associated Antigen (TAA)), a hinge region, a transmembrane region and an intracellular domain. CAR-T cell immunotherapy is considered to be one of the most promising methods to conquer tumors. CAR-T cells use genetic modification methods to make T cells express CAR proteins, and such CAR protein has the ability to recognize intact proteins on the membrane surface without depending on antigen presentation, thereby causing T cell activation and functional effects.

In 2021, Bristol-Myers Squibb and Bluebird Bio announced together that the U.S. Food and Drug Administration (FDA) has approved their CAR-T cell therapy targeting BCMA (bb2121), which are used for adult patients with relapsed or refractory multiple myeloma after 4-line treatment (including immunomodulators, proteasome inhibitors, and antibody drug therapy). This is the world's first CAR-T cell therapy targeting BCMA. It is of practical significance to develop more and more effective cell therapies targeting BCMA.

### SUMMARY OF THE INVENTION

The present application provides a BCMA-targeting chimeric antigen receptor and use thereof. The inventors have used multiple scFvs targeting BCMA to construct chimeric antigen receptor expression vectors and prepare CAR-T cells targeting BCMA. They also have verified that BCMA CAR-T cells have good anti-tumor function at the cellular level and animal level, and identified optimal BCMA-targeting chimeric antigen receptors.

A BCMA-targeting chimeric antigen receptor, comprising an extracellular antigen recognition domain, a hinge region, a transmembrane region and an intracellular domain; wherein the extracellular antigen recognition domain comprises an anti-BCMA scFv antibody, the amino acid sequences of VH complementarity determining regions CDR1, CDR2, and CDR3 of the scFv antibody respectively comprise amino acid sequences as shown in SEQ ID NO: 1, SEQ ID NO: 2, and SEQ ID NO: 3, and the amino acid sequences of the VL complementarity determining regions CDR1, CDR2, and CDR3 of the scFv antibody respectively comprise amino acid sequences as shown in SEQ ID NO: 4, SEQ ID NO: 5, and SEQ ID NO: 6.

In certain embodiments of the above-mentioned chimeric antigen receptor, the scFv antibody is a humanized antibody; optionally, VH sequence of the scFv antibody comprises the amino acid sequence as shown in SEQ ID NO: 7, and VL sequence of the scFv antibody comprises the amino acid sequence as shown in SEQ ID NO: 8.

In certain embodiments of the above-mentioned chimeric antigen receptor, the scFv antibody is a rabbit-derived antibody; optionally, VH sequence of the scFv antibody comprises the amino acid sequence as shown in SEQ ID NO: 9, and VL sequence of the scFv antibody comprises the amino acid sequence as shown in SEQ ID NO: 10.

In certain embodiments of the above-mentioned chimeric antigen receptor, the scFv antibody has a linker region between VH and VL, and the linker region is selected from one or more of the following sequences: SEQ ID NOs: 37-39.

In certain embodiments of the above-mentioned chimeric antigen receptor, the sequence of the scFv antibody is shown in SEQ ID NO: 11 or SEQ ID NO: 12.

In certain embodiments of the above-mentioned chimeric antigen receptor, the hinge region is derived from one or more of IgG1, IgG4, CD4, CD7, CD28, CD84, and CD8α.

In certain embodiments of the above-mentioned chimeric antigen receptor, the transmembrane region is derived from one or more of CD3, CD4, CD7, CD8α, CD28, CD80, CD86, CD88, 4-1BB, CD152, OX40, and Fc70.

In certain embodiments of the above-mentioned chimeric antigen receptor, the intracellular domain comprises an intracellular signal transduction region; optionally, the intracellular domain further comprises a costimulatory signal transduction region.

In certain embodiments of the above-mentioned chimeric antigen receptor, the intracellular signal transduction region is derived from one or more of CD3ζ, CD3γ, CD3δ, CD3ε, CD5, CD22, CD79a, CD79b, FcRy, FcRβ, CD66d, DAP10, DAP12, and Syk.

In certain embodiments of the above-mentioned chimeric antigen receptor, the costimulatory signal transduction region is derived from one or two or more of CD2, CD3, CD7, CD27, CD28, CD30, CD40, CD83, CD244, 4-1BB, OX40, LFA-1, ICOS, LIGHT, NKG2C, NKG2D, DAP10, B7-H3, and MyD88.

In certain embodiments of the above-mentioned chimeric antigen receptor, the chimeric antigen receptor further comprises a guiding peptide located at the N-terminus of the amino acid sequence of the chimeric antigen receptor; optionally, the guiding peptide is derived from CD8α.

In certain embodiments of the above-mentioned chimeric antigen receptor, the extracellular antigen recognition domain further comprises a scFv antibody against one of the following targets: CD138, NKG2D, CD38, CD19, SLAMF7, CD70, CD44v6, and Lewis Y.

The present application also provides an isolated nucleic acid molecule comprising a nucleic acid sequence encoding the above-mentioned chimeric antigen receptor.

The present application also provides a vector comprising the above-mentioned isolated nucleic acid molecule.

In certain embodiments of the above-mentioned vector, the vector is an expression vector; in some embodiments, the vector is a viral vector; in certain embodiments, the vector is a lentiviral vector.

The present application also provides an engineered immune effector cell comprising the above-mentioned chimeric antigen receptor, the above-mentioned isolated nucleic acid molecule or the above-mentioned vector.

In certain embodiments of the above-mentioned immune effector cell, the immune effector cell is selected from one or more of T lymphocyte, natural killer cell (NK cell), peripheral blood mononuclear cell (PBMC cell), pluripotent stem cell, T cell differentiated from pluripotent stem cell, NK cell differentiated from pluripotent stem cell, induced pluripotent stem cell (iPSC), T cell differentiated from induced pluripotent stem cell (iPSC-T), NK cell differentiated from induced pluripotent stem cell (iPSC-NK) and embryonic stem cell.

In certain embodiments of the above-mentioned immune effector cell, the immune effector cell is T lymphocyte; optionally, the source of the T lymphocyte is autologous T lymphocyte or allogeneic T lymphocyte.

The present application also provides a pharmaceutical composition comprising the above-mentioned engineered immune effector cell, and pharmaceutically acceptable adjuvant.

In certain embodiments of the above-mentioned pharmaceutical composition, the pharmaceutically acceptable adjuvant comprises a protective agent.

In certain embodiments of the above-mentioned pharmaceutical composition, the pharmaceutically acceptable adjuvant comprises a cell cryopreservation solution.

In certain embodiments, the above-mentioned pharmaceutical composition is an intravenous injection.

The present application also provides use of the above-mentioned chimeric antigen receptor, the above-mentioned nucleic acid molecule, the above-mentioned vector or the above-mentioned immune effector cell in the preparation of a medicament for treating a disease or condition associated with the expression of BCMA.

In certain embodiments of the above-mentioned use, the disease or condition associated with the expression of BCMA is cancer; optionally, the cancer is multiple myeloma; more optionally, the cancer is refractory or relapsed multiple myeloma.

In certain embodiments of the above-mentioned use, the disease or condition associated with expression of BCMA may be an autoimmune disease.

In certain embodiments of the above-mentioned use, the autoimmune disease may be selected from the group consisting of systemic lupus erythematosus, rheumatoid arthritis, idiopathic thrombocytopenic purpura, myasthenia gravis, or autoimmune hemolytic anemia.

In certain embodiments of the above-mentioned use, the medicament is an intravenous injection.

The present application also provides a method for treating a disease or condition associated with the expression of BCMA, including the following steps: administering an effective amount of the above-mentioned immune effector cell or the above-mentioned pharmaceutical composition to a subject having a need to treat a disease or condition associated with the expression of BCMA.

In certain embodiments of the above-mentioned method, the disease or condition associated with the expression of BCMA is cancer; optionally, the cancer is multiple myeloma; more optionally, the cancer is refractory or relapsed multiple myeloma.

In certain embodiments of the above-mentioned method, the disease or condition associated with expression of BCMA may be an autoimmune disease.

In certain embodiments of the above-mentioned method, the autoimmune disease may be selected from the group consisting of systemic lupus erythematosus, rheumatoid arthritis, idiopathic thrombocytopenic purpura, myasthenia gravis, or autoimmune hemolytic anemia.

In certain embodiments of the above-mentioned method, the administration method is intravenous injection.

In certain embodiments of the above-mentioned method, the administration method is to administer an effective amount of the above-mentioned immune effector cell or the above-mentioned pharmaceutical composition to a subject in a single injection.

In certain embodiments of the above-mentioned method, the effective amount of the above-mentioned immune effector cell or the above-mentioned pharmaceutical composition is at a dose of 1×10⁵ to 1×10⁷ cells/kg.

The present application also provides the above-mentioned immune effector cell or the above-mentioned pharmaceutical composition, for use in the treatment of a disease or condition associated with the expression of BCMA.

In certain embodiments of the above-mentioned immune effector cell or the above-mentioned pharmaceutical composition, the disease or condition associated with the expression of BCMA is cancer; optionally, the cancer is multiple myeloma; more optionally, the cancer is refractory or relapsed multiple myeloma.

In certain embodiments of the above-mentioned immune effector cell or the above-mentioned pharmaceutical composition, the disease or condition associated with the expression of BCMA may be an autoimmune disease.

In certain embodiments of the above-mentioned immune effector cell or the above-mentioned pharmaceutical composition, the autoimmune disease can be selected from the group consisting of systemic lupus erythematosus, rheumatoid arthritis, idiopathic thrombocytopenic purpura, myasthenia gravis or autoimmune hemolytic anemia.

### BRIEF DESCRIPTION OF THE DRAWINGS

Fig. 1 takes BY-02G as an example to show the basic structure of the CAR molecule.
Fig. 2 shows the expression of BCMA CAR molecules on the surface of CAR-T cells in Example 2, wherein: 01G-CAR-05G-CAR represents T cells that transduce with CAR, and UTD represents T cells that do not transduce with CAR. In each figure, the left peak represents UTD, and the right peak represents 01G-CAR, 02G-CAR, 03G-CAR, 04G-CAR, and 05G-CAR, respectively.
Fig. 3A shows the number of CAR positive T cells in each group D0-D6 in multiple rounds of antigen stimulation experiments in Example 3; Fig. 3B shows the number of CAR positive T cells in each group D8-D14 in multiple rounds of antigen stimulation experiments in Example 3; Fig. 3C shows the CAR MFI of each group D2-D6 in the multiple rounds of antigen stimulation experiments in Example 3; Fig. 3D shows the CAR MFI of D8-D14 in the multiple rounds of antigen stimulation experiments in Example 3.
Fig. 4A shows the proportion of CAR positive T cells from D0 to D6 in multiple rounds of antigen stimulation experiments in Example 3; Fig. 4B shows the proportion of CAR positive T cells from D8 to D14 in multiple rounds of antigen stimulation experiments in Example 3.
Fig. 5 shows the expression rates of cell surface immune checkpoint proteins PD1, Lag3, and Tim 3 after multiple rounds of antigen stimulation of BCMA CAR-T cells in Example 4. For cells in each group, from left to right, they represent the expression rates of PD1, Lag3, and Tim 3.
Fig. 6 shows the Annexin V positive proportion of CAR positive cells after multiple rounds of antigen stimulation of BCMA CAR-T cells in Example 5.
Fig. 7 shows the release of cytokine IFN-y after multiple rounds of antigen stimulation of BCMA CAR-T cells in Example 6. For K562 cells and MM. 1S cells, from left to right, they are the cytokine IFN-y release levels of nontransduced CAR T cells, BY-01G cells, BY-02G cells, BY-04G cells and BY-05G cells.
Fig. 8A shows the tumor volume change curve of each group in the subcutaneous tumor-bearing animal experiment of RPMI8226 cells in Example 7; Fig. 8B shows the average fluorescence imaging signal intensity change curve of each group in the tail vein tumor-bearing animal experiment of MM. 1S cells in immunodeficient mice in Example 7.
Fig. 9A shows the survival curves of the low-dose BY-02G group and the bb2121 group in Example 7; Fig. 9B shows the survival curves of the middle-dose BY-02G group and the bb2121 group in Example 7.
Fig. 10 shows the average weight change curve of each group in Example 8.
Fig. 11 shows the specific recognition of BCMA by BCMA CAR-T cells in Example 9, the first row of each target cell in the figure shows the expression of BCMA protein on the surface of the target cell, and the second row shows the expression of CD 107a on the surface of CD8-positive T cells.
Fig. 12 shows the specific recognition of BCMA by BCMA scFv in Example 9.

### DETAILED DESCRIPTION OF THE INVENTION

The embodiments of the present invention will be illustrated with the following specific examples. Those familiar with this technology can easily understand other advantages and effects of the present invention from the disclosures in the present description.

The present application is further described below: in the present invention, unless otherwise stated, the scientific and technical terms used herein have the meanings commonly understood by those skilled in the art. Furthermore, the terms and laboratory procedures related to protein and nucleic acid chemistry, molecular biology, cell and tissue culture, microbiology, and immunology used herein are terms and routine procedures widely used in the corresponding fields. Meanwhile, in order to better understand the present invention, definitions and explanations of relevant terms are provided below.

In the present application, the term "Chimeric Antigen Receptor (CAR)" is the core component of CAR cell therapy, which may include an extracellular antigen recognition domain (for example, the portion that binds to Tumor-Associated Antigen (TAA), a hinge region, a transmembrane region and an intracellular domain. CAR-T (Chimeric Antigen Receptor T) cellular immunotherapy is considered to be one of the most promising methods to conquer tumors. CAR-T cells use genetic modification methods to make T cells express CAR proteins, and such CAR protein has the ability to recognize intact proteins on the membrane surface without depending on antigen presentation, thereby causing T cell activation and functional effects.

In the present application, the term "extracellular antigen recognition domain" refers to the Antigen Recognition Domain (ARD). The reason why CAR cell therapy products (such as CAR-T cells) can specifically recognize and/or bind to target antigens expressed by tumor cells depends on the extracellular antigen recognition domain. So far, the antigen recognition domain has been derived from Single Chain Variable Fragment (abbreviated as scFv) of an antibody, or from receptor-ligand interactions, TCR mimics, Variable Lymphocyte Receptors (VLR), and the most common source is scFv antibody. Unless otherwise specified in the present application, scFv antibody refers to scFv antibody targeting BCMA. scFv antibody comprises one, two, or more than two antibody heavy chain variable regions (VH) and one, two, or more than two light chain variable regions (VL), wherein VH and VL are connected by a peptide chain, such as the linker sequence GSTSGSGKPGSGEGSTKG consisting of 18 amino acids. There are a small number of amino acid residues in the antibody variable region that are particularly strongly changed. The regions where the composition and arrangement of these amino acid residues are more likely to vary are called hypervariable regions (HVR); in the V regions of the L chain and H chain, each has three hypervariable regions, which are also called complementarity determining regions (CDRs) because their spatial structure can form precise complementarity with the antigenic determinants. In antibodies, common rules for dividing CDR include Kabat, AbM, Chothia, Contact, and IMGT. These rules are well-known to those skilled in the art. When applying the website that implements these rules, once the VH and VL sequences are entered and the corresponding rules are selected, CDR sequences based on different rules can be obtained. Those skilled in the art should understand that the protection scope of the present application covers combinations of CDR sequences obtained by analyzing using different rules. In the present application, CDR is divided through IMGT rules.

In the present application, the term "specific recognition and/or binding" refers to the recognition and/or binding between CAR and specific targets, CAR binding to this target with greater affinity, avidity, more easily, and/or with greater duration than CAR binding to other targets.

In the present application, the term "humanized antibody" is also referred to an antibody that has been humanized modified, which is prepared by transplanting the complementarity determining regions (CDR) of non-human mammalian antibody, such as mouse antibody, rat antibody, and rabbit antibody, into the CDRs of human antibody. The conventional recombinant DNA technology for preparing humanized antibodies is known (e.g., WO96/02576). For example, in the case that the CDR is obtained from a rabbit antibody, primers can be synthesized (the corresponding primers can be obtained by referring to the method described in WO98/13388) and primers can be used to connect the CDR of the rabbit antibody to the framework region (FR) of the human antibody. For the human antibody FR linked to the CDR, those that enable the CDR region to form a good antigen-binding site should be selected.

In the present application, the term "hinge region" refers to the linker segment between the extracellular antigen recognition domain and the transmembrane domain. This region allows the CAR to recognize the antigen by providing a certain range of activity to the antigen recognition domain. The hinge regions currently used are mainly derived from one or more of IgG1, IgG4, CD4, CD7, CD28, CD84, and CD8α. In addition, the typical hinge region also contains some residues that participate in CAR dimerization and contribute to enhance antigen sensitivity.

In the present application, "transmembrane region" refers to the transmembrane domain that connects the intracellular and extracellular components of the CAR structure. Different transmembrane domains can affect the expression and stability of CAR to some extent, but they are not directly involved in signal transduction, and they improve downstream signal transduction by interactions. The transmembrane region may be derived from one or more of CD3, CD4, CD7, CD8α, CD28, CD80, CD86, CD88, 4-1BB, CD152, OX40, and Fc70.

In present application, the term "intracellular domain" comprises a intracellular signal transduction region and may further comprise a costimulatory signal transduction region.

In the present application, the term "intracellular signal transduction region" refers to the activation of at least one normal effector function of immune effector cells responsible for expressing CAR. The intracellular signal transduction region may be derived from one or more of CD3ζ, CD3γ, CD3δ, CD3ε, CD5, CD22, CD79a, CD79b, FcRy, FcRβ, CD66d, DAP10, DAP12, and Syk.

In the present application, the term "costimulatory signal transduction region" exists because in addition to stimulation by antigen-specific signal, many immune effector cells require costimulation to promote cell proliferation, differentiation, and survival, as well as effector function of activated cells. In some examples, the CAR may further comprise one or more costimulatory signal transduction region, wherein the costimulatory signal transduction region may be derived from one, two or more of CD2, CD3, CD7, CD27, CD28, CD30, CD40, CD83, CD244, 4-1BB, OX40, LFA-1, ICOS, LIGHT, NKG2C, NKG2D, DAP10, B7-H3 and MyD88.

In the present application, the term "isolated" generally means those obtained from the natural state by artificial means. If an "isolated" substance or component occurs in nature, it may be that the natural environment in which it is located has changed, or that the substance has been separated from its natural environment, or both. For example, a certain unisolated polynucleotide or polypeptide naturally exists in a living animal, and the high purity of the same polynucleotide or polypeptide isolated from this natural state is called isolated. The term "isolated" does not exclude substances that have been artificially or synthetically obtained from their natural state by artificial means, nor does it exclude the presence of other impure substances that do not affect the activity of the substance.

In the present application, the term "guiding peptide" refers to the short peptide before the extracellular antigen recognition domain (such as the scFv sequence), whose function is to guide the recombinant protein synthesized in the cell to be exported to the outside of the cell. Commonly used guiding peptides include human CD8α signal peptide or human GM-CSF receptor α signal peptide.

In the present application, one of the key factors that determine the therapeutic effect of CAR immune cells is the selection of tumor target antigens. In the present application, the term "BCMA" refers to B cell maturation antigen, and is a member of the tumor necrosis factor receptor superfamily. Human BCMA is expressed almost exclusively on plasma cells and multiple myeloma cells. BCMA may be a suitable tumor antigen target for immunotherapeutic agent against multiple myeloma. However, due to the heterogeneity of specific antigens on the surface of multiple myeloma cells, the selection of its antigen target is not necessarily a single one. By selecting appropriate targets, the anti-tumor activity of CAR-T cells can be optimized. Therefore, the extracellular antigen recognition domain may further comprise extracellular antigen recognition domain (such as scFv antibody) targeting any of the following targets: CD138, NKG2D, CD38, CD19, SLAMF7, CD70, CD44v6, and Lewis Y. For example, in dual-target CAR-T products, the extracellular antigen recognition domain comprises scFv sequences against two targets. scFv antibody against a single target comprises the antibody heavy chain variable region (VH) and the light chain variable region (VL), wherein VH and VL are linked through a linker sequence. scFv antibody against two or more targets comprises VH regions and VL regions targeting different targets, wherein different regions are also linked directly or indirectly through linker sequences. Their arrangement can be in any of the following forms: Target 1 VL-Target 1 VH-Target 2 VL-Target 2 VH, Target 2 VL-Target 2 VH-Target 1 VL-Target 1 VH, Target 1 VL-Target 2 VL-Target 2 VH-Target 1 VH, Target 2 VL-Target 1 VL-Target 1 VH-Target 2 VH, wherein the above "-" represents linking through the linker sequence.

In the present application, the term "isolated nucleic acid molecule" generally refers to an isolated form of a nucleotide, deoxyribonucleotide or ribonucleotide of any length, which may be isolated from its natural environment or a synthetic analog.

In the present application, when performing CAR gene transduction/transfection and target gene expression, gene transduction/transfection methods mainly include viral and non-viral methods. For example: the methods through gamma retroviral vectors, lentiviral vectors, adenovirus-associated viral vectors, plasmid DNA-dependent vectors, transposon-dependent gene transfer, and mRNA-mediated gene transduction.

The term "vector" generally refers to a nucleic acid delivery vehicle into which a polynucleotide encoding a protein can be inserted and the protein can be expressed. The vector can be transformed, transduced or transfected into the host cell so that the genetic material elements it carries can be expressed in the host cell. For example, vectors include: plasmids; phagemids; cosmids; artificial chromosomes such as yeast artificial chromosomes (YAC), bacterial artificial chromosomes (BAC) or P1-derived artificial chromosomes (PAC); phages such as lambda phage or M13 phage and animal viruses, etc. The types of animal viruses used as vectors include retroviruses (including lentiviruses), adenoviruses, adeno-associated viruses, herpesviruses (such as herpes simplex virus), poxviruses, baculoviruses, papillomaviruses, and papillomavirus (such as SV40). A vector may contain a variety of elements that control expression, including promoter sequences, transcription initiation sequences, enhancer sequences, selection elements, and reporter genes. In addition, the vector may also contain a replication origin. Vectors may also contain components that assist them in entering into cells, such as viral particles, liposomes, or protein coats, but not just these substances. The term "transposon" refers to discontinuous DNA segments that have the ability to migrate and carry genetic information between chromosomal sites, such as the Sleeping Beauty SB system and the PB system derived from lepidopteran insects. In some examples, mRNA can also be transduced into T cells by means of electrotransformation.

In the present application, the term "immune effector cell" generally refers to cell that participates in immune response, for example, the cell that promotes immune effector response. Immune effector cell can be selected from the following groups: one or more of T lymphocyte, natural killer cell (NK cell), peripheral blood mononuclear cell (PBMC cell), pluripotent stem cell, T lymphocyte differentiated from pluripotent stem cell, NK cell differentiated from pluripotent stem cell, induced pluripotent stem cell (iPSC), T cell differentiated from induced pluripotent stem cell (iPSC-T), NK cell differentiated from induced pluripotent stem cell (iPSC-NK), and embryonic stem cell.

In the present application, the term "pharmaceutical composition" generally refers to a pharmaceutical composition suitable for administration to a patient, which may comprise the immune effector cell as described in the present application, and may further comprise one or more pharmaceutically acceptable adjuvant, such as: one or more of carrier, protective agent, stabilizer, excipient, diluent, solubilizer, surfactant, emulsifier and preservative. In some examples, pharmaceutically acceptable adjuvant comprises a protective agent, such as cell cryopreservation solution. In some examples, the pharmaceutical composition of the present application is a cell suspension or cryopreserved cell thereof.

In the present application, the term "subject" generally refers to a human or non-human animal, including but not limited to mouse, rat, cat, dog, rabbit, horse, pig, cow, sheep, or monkey.

In the present application, the term "comprise/comprising" generally refers to the inclusion of explicitly specified features, but not the exclusion of other elements.

In the present application, the term "about" generally refers to a range of fluctuations above or below the specified value that is acceptable to those skilled in the art, such as: changes within the range of ±0.5%-10%, such as changes within the range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5% or 10% above or below the specified value.

### Chimeric antigen receptor, nucleic acid, vector, immune effector cell, pharmaceutical composition

On the one hand, the present application provides a BCMA-targeting chimeric antigen receptor, comprising an extracellular antigen recognition domain, a hinge region, a transmembrane region and an intracellular domain; wherein the extracellular antigen recognition domain comprises an anti-BCMA scFv antibody, the amino acid sequences of VH complementarity determining regions CDR1, CDR2, and CDR3 of the scFv antibody respectively comprise amino acid sequences as shown in SEQ ID NO: 1, SEQ ID NO: 2, and SEQ ID NO: 3, and the amino acid sequences of the VL complementarity determining regions CDR1, CDR2, and CDR3 of the scFv antibody respectively comprise amino acid sequences as shown in SEQ ID NO: 4, SEQ ID NO: 5, and SEQ ID NO: 6.

In some examples, the scFv antibody is a humanized antibody; optionally, VH sequence of the scFv antibody comprises the amino acid sequence as shown in SEQ ID NO: 7, and VL sequence of the scFv antibody comprises the amino acid sequence as shown in SEQ ID NO: 8.

In some examples, the scFv antibody is a rabbit-derived antibody; optionally, VH sequence of the scFv antibody comprises the amino acid sequence as shown in SEQ ID NO: 9, and VL sequence of the scFv antibody comprises the amino acid sequence as shown in SEQ ID NO: 10.

In some examples, the scFv antibody has a linker region between VH and VL, and the linker region is selected from one or more of the following sequences: SEQ ID NOs: 37-39.

In some examples, the sequence of the scFv antibody is shown in SEQ ID NO: 11 or SEQ ID NO: 12.

In some examples, the present application also includes the substitution, deletion, addition and/or insertion of one or more amino acids in the amino acid sequence of any of the above-mentioned chimeric antigen receptor; and their activity equivalent to any of the above-mentioned chimeric antigen receptor; optionally, the substitution is a conservative substitution. Those skilled in the art know that during the process of humanization, the amino acids in the scFv FR region can be substituted so that the CDR region of the modified antibody can retain a suitable antigen-binding site. Therefore, the present application certainly includes amino acid sequences obtained by humanizing the FR region in scFv based on the above-mentioned CDR, which are different from those as shown in SEQ ID NO: 7, SEQ ID NO: 8, SEQ ID NO: 9, or SEQ ID NO: 10. Moreover, those skilled in the art also know that in the process of humanization, in order to ensure that the CDR region of the modified antibody can retain a suitable antigen-binding site, if necessary, 1, 2, 3, or no more than 10% of amino acid sequences in CDR may be substituted, deleted, added, and/or inserted, which are also included in the present application.

In some examples, the hinge region is derived from one or more of IgG1, IgG4, CD4, CD7, CD28, CD84, and CD8α; optionally, the amino acid sequence of the hinge region is derived from CD8α; more optionally, the amino acid sequence of the hinge region comprises the amino acid sequence as shown in SEQ ID NO: 13.

In some examples, the transmembrane region is derived from one or more of CD3, CD4, CD7, CD8α, CD28, CD80, CD86, CD88, 4-1BB, CD152, OX40, and Fc70; optionally, the amino acid sequence of the transmembrane region is derived from CD8α; more optionally, the amino acid sequence of the transmembrane region comprises the amino acid sequence as shown in SEQ ID NO: 14.

In some examples, the intracellular domain comprises an intracellular signal transduction region; optionally, the intracellular domain comprises a costimulatory signal transduction region; more optionally, the intracellular signal transduction region is derived from one or more of CD3ζ, CD3γ, CD3δ, CD3ε, CD5, CD22, CD79a, CD79b, FcRy, FcRβ, CD66d, DAP10, DAP12 and Syk; even more optionally, the intracellular signal transduction region is derived from CD3ζ, for example, the amino acid sequence of the intracellular signal transduction region comprises the amino acid sequence as shown in SEQ ID NO: 15.

In some examples, the costimulatory signal transduction region is derived from one, two or more of CD2, CD3, CD7, CD27, CD28, CD30, CD40, CD83, CD244, 4-1BB, OX40, LFA-1, ICOS, LIGHT, NKG2C, NKG2D, DAP10, B7-H3, and MyD88; optionally, the costimulatory signal transduction region is derived from CD28 or 4-1BB; more optionally, the amino acid sequence of the costimulatory signal transduction region comprises the amino acid sequence as shown in SEQ ID NO: 16.

In some examples, the chimeric antigen receptor further comprises a guiding peptide located at the N-terminus of the amino acid sequence of the chimeric antigen receptor; optionally, the guiding peptide is derived from CD8α; more optionally, the amino acid sequence of the guiding peptide comprises the amino acid sequence as shown in SEQ ID NO: 17.

In some examples, the chimeric antigen receptor of the present invention comprises the amino acid sequence as shown in SEQ ID NO: 28.

In some examples, the extracellular antigen recognition domain only comprises scFv antibody targeting the single target of BCMA.

In some examples, the extracellular antigen recognition domain further comprises a scFv antibody against any of the following targets: CD138, NKG2D, CD38, CD19, SLAMF7, CD70, CD44v6, and Lewis Y.

On the other hand, the present application also provides an isolated nucleic acid molecule comprising the nucleic acid sequence encoding the above-mentioned chimeric antigen receptor.

On the other hand, the present application provides an isolated nucleic acid molecule encoding a chimeric antigen receptor, wherein the nucleic acid molecule comprises the nucleotide sequence as shown in SEQ ID NO: 18, or a nucleotide sequence having at least 70%, at least 75%, at least 80%, at least 85%, at least 90%, at least 95%, at least 96%, at least 97%, at least 98%, or at least 99% sequence identity to the nucleotide sequence as shown in SEQ ID NO: 18 and encoding the same chimeric antigen receptor. This sequence identity is caused due to the wobble (degeneracy) of the third base of the nucleic acid codon. Optionally, the nucleic acid molecule comprises the nucleotide sequence as shown in SEQ ID NO: 33.

On the other hand, the present application also provides a vector comprising the above-mentioned isolated nucleic acid molecule. Vectors include: plasmids; phagemids; cosmids; artificial chromosomes such as yeast artificial chromosomes (YAC), bacterial artificial chromosomes (BAC) or P1-derived artificial chromosomes (PAC); phages such as lambda phage or M13 phage and animal viruses, etc. The types of animal viruses used as vectors include retroviruses (including lentiviruses), adenoviruses, adeno-associated viruses, herpesviruses (such as herpes simplex virus), poxviruses, baculoviruses, papillomaviruses, and papillomavirus (such as SV40).

In some examples, the vector is an expression vector; optionally, the vector is a viral vector; more optionally, the vector is a lentiviral vector.

On the other hand, the present application also provides an engineered immune effector cell, comprising the above-mentioned chimeric antigen receptor, the above-mentioned isolated nucleic acid molecule, or the above-mentioned vector.

In some examples, the immune effector cell is selected from one or more of T lymphocyte, natural killer cell (NK cell), peripheral blood mononuclear cell (PBMC cell), pluripotent stem cell, and T cell differentiated from pluripotent stem cell, NK cell differentiated from pluripotent stem cell, and embryonic stem cell.

In some examples, the immune effector cell is T lymphocyte; optionally, the source of the T lymphocyte is autologous T lymphocyte or allogeneic T lymphocyte.

In some examples, the chimeric antigen receptor as described in the present application can be expressed on the surface of the immune effector cell.

On the other hand, the present application also provides a pharmaceutical composition, comprising the above-mentioned engineered immune effector cell, and pharmaceutically acceptable adjuvant. The pharmaceutically acceptable adjuvant include: one or more of carrier, protective agent, stabilizer, excipient, diluent, solubilizer, surfactant, emulsifier, and preservative.

In some examples, the pharmaceutically acceptable adjuvant comprises a protective agent, such as cell cryopreservation solution.

In some examples, the pharmaceutical composition is a cell suspension or cryopreserved cell thereof.

In some examples, the pharmaceutical composition is an intravenous injection.

### Preparation method and use

On the other hand, the present application also provides a method for preparing immune effector cell, including the following steps: transducing the vector as described in the present application into the immune effector cell.

In some examples, the immune effector cell is selected from one or more of T lymphocyte, natural killer cell (NK cell), peripheral blood mononuclear cell (PBMC cell), pluripotent stem cell, and T cell differentiated from pluripotent stem cell, NK cell differentiated from pluripotent stem cell, and embryonic stem cell.

In some examples, the immune effector cell is T lymphocyte; optionally, the source of the T lymphocyte is autologous T lymphocyte or allogeneic T lymphocyte.

On the other hand, the present application also provides use of the chimeric antigen receptor, the nucleic acid molecule, the vector and/or the immune effector cell as described in the present application for preparing a medicament, wherein the medicament is used for the treatment of a disease or condition associated with the expression of BCMA.

On the other hand, the present application also provides a method for treating a disease or disorder associated with the expression of BCMA, including administering an effective dose of the chimeric antigen receptor, nucleic acid molecule, vector, and/or immune effector cell as described the present application to a subject having a need to treat a disease or condition associated with BCMA expression.

In some examples, the administration can be carried out in different ways, such as intravenous, intratumoral, intraperitoneal, subcutaneous, intramuscular, topical or intradermal administration. For example, the method of administration may be administered to a subject by intravenous injection. In some examples, an effective dose of immune effector cell or the pharmaceutical composition can be administered to a subject in a single time, or in divided doses within a certain period, such as once a week, once every two weeks, once every three weeks, once every four weeks, once a month, once every 3 months, or once every 3-6 months.

In some examples, the doses for administration may vary for different indications; and the doses for administration may also vary depending on the severity of the patient' s condition. The dose may be administered in a range of 1×10⁵ CAR positive T cells/kg to 1×10⁷ CAR positive T cells/kg, for example, 1×10⁵ CAR positive T cells/kg to 1×10⁶ CAR positive T cells/kg, 1×10⁶ CAR positive T cells/kg to 1×10⁷ CAR positive T cells/kg. The dose for administration may also be measured by the total dose for administration, for example: the total dose for administration does not exceed 5×10⁸ CAR positive T cells. In the present application, the dose for administration is counted based on CAR positive T cells, which will not be described again in the specific examples.

In some examples, the subject may include human and non-human animal. For example, the subject may include, but not limited to, mouse, rat, cat, dog, horse, pig, cow, sheep, rabbit, or monkey.

On the other hand, the present application also provides the chimeric antigen receptor, the nucleic acid molecule, the vector and/or the immune effector cell, for use in the treatment of a disease or condition associated with the expression of BCMA.

In some examples, the disease or condition associated with expression of BCMA may include non-solid tumor; optionally, the non-solid tumor is a hematological tumor.

In some examples, the disease or condition associated with expression of BCMA may include multiple myeloma.

In some examples, the multiple myeloma is relapsed or refractory multiple myeloma.

In some examples, the disease or condition associated with expression of BCMA may be an autoimmune disease.

In some examples, the autoimmune disease may be selected from the following: systemic lupus erythematosus, rheumatoid arthritis, idiopathic thrombocytopenic purpura, myasthenia gravis, or autoimmune hemolytic anemia.

Without wishing to be bound by any theory, the following examples are only described to illustrate the chimeric antigen receptor, the immune effector cell, the preparation method and use of the present application, and are not used to limit the scope thereof. The examples do not include detailed descriptions of traditional methods, such as those used to construct vectors and plasmids, methods of inserting genes encoding proteins into such vectors and plasmids, or methods of introducing plasmids into host cells. Such methods have been well-known to those of ordinary skill in the art, and described in many publications, including Sambrook, J., Fritsch, E. F. and Maniais, T. (1989) Molecular Cloning: A Laboratory Manual, 2nd edition, Cold spring Harbor Laboratory Press.

### Examples

### Example 1. Obtaining BCMA CAR-T cells

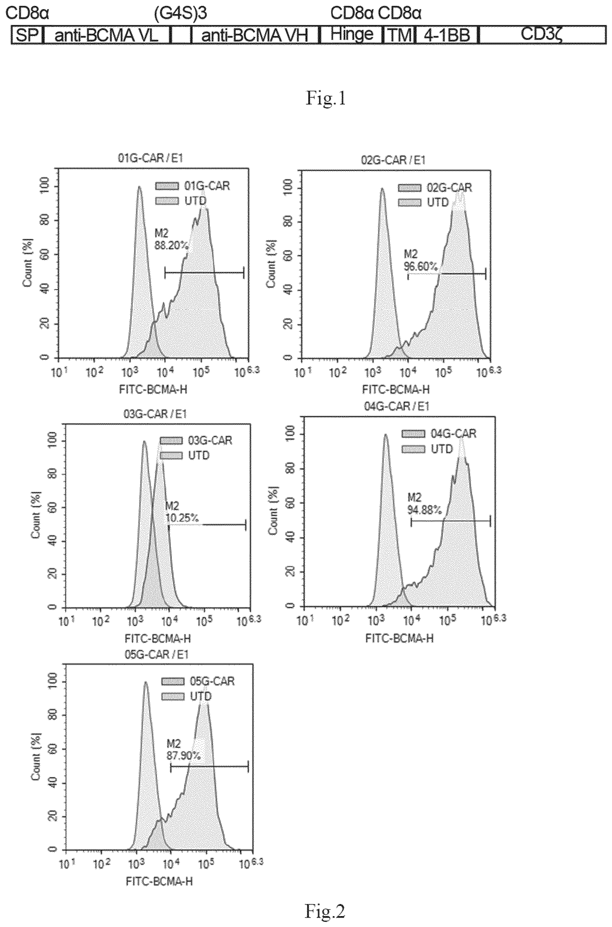

Five BCMA-specific scFv sequences (the amino acid sequences of their scFvs are shown in SEQ ID NO: 19, SEQ ID NO: 11, SEQ ID NO: 20, SEQ ID NO: 21, and SEQ ID NO: 22, respectivley, the amino acid sequences of these 5 scFvs are also used as an abbreviations with the number of BY-01G, BY-02G, BY-03G, BY-04G, and BY-05G in the present application. The nucleotide sequences encoding the above-mentioned BY-01G to BY-05G scFvs are shown in SEQ ID NO: 23, SEQ ID NO: 18, SEQ ID NO: 24, SEQ ID NO: 25, and SEQ ID NO: 26, respectively) have been obtained. Since functional verification of scFvs at the protein level cannot reflect their functions at the cellular level, candidate BCMA scFv sequences on the second-generation CAR structures were selected. The schematic diagram of the structure of CAR is shown in Fig. 1. The following structures were used in this example: CD8α guiding chain as the signal peptide, BCMA scFv as the extracellular tumor antigen recognition region, the structure of CD8α as the hinge region and transmembrane region, 4-1BB as the intracellular costimulatory signal, and CD3ζ as the T cell activation signal.

### 1. Construction of lentiviral vectors

CAR structural fragments containing BY-01G-BY-05G scFvs described in the present application (their amino acid sequences are shown in SEQ ID NO: 27, SEQ ID NO: 28, SEQ ID NO: 29, SEQ ID NO: 30, and SEQ ID NO: 31, respectivley, and the nucleotide sequences encoding the above-mentioned CAR structural fragments are shown in SEQ ID NO: 32, SEQ ID NO: 33, SEQ ID NO: 34, SEQ ID NO: 35, and SEQ ID NO: 36, respectively) were artificially synthesized, respectively, and were respectively constructed into resistance-modified empty lentiviral vectors (manufacturer: SBI Corporation, catalog number: CD500-CD800, as described in Example 1 of WO2021/121227, which was resistance-modified) to obtain the CAR expression vectors, then the CAR expression vectors and three packaging plasmids were transfected into 293T cells. After collection and purification, functional lentiviral vectors were obtained. The three packaging plasmids were PMD2.0G (purchased from Biovector Corporation, catalog number: Biovector 012259), pMDLg-pRRE (purchased from Biovector Corporation, catalog number: Biovector 012251), and pRSV-Rev (purchased from Biovector Corporation, catalog number: Biovector 012253), respectively.

### 2. Preparation the corresponding 5 types of BCMA CAR-T cells through lentiviral transduction

The transduction experiment was carried out according to conventional methods known to those skilled in the art. The steps of transduction were briefly described as follows:

### 1. Sorting of T cells

Peripheral blood mononuclear cells (PBMC) were isolated from the subject's apheresis cells, and then T cells were sorted from the PBMC cells.

### 2. Activation of the T cells

The isolated T cells were resuspended with complete lymphocyte culture medium (X-VIVO15 medium + 5% FBS + 300 IU/ml IL-2 or X-VIVO15 medium + 5% FBS + 5 ng/ml IL-15 + 10 ng/ml IL-7) to give a final concentration of (1~2)×10⁶ cells/ml, and 5 to 10 µl of CD3/CD28 stimulation magnetic beads were added. The mixture was well mixed, and placed in an incubator for culture for at least 24 h under the culture condition of 37°C+5% CO₂.

### 3. Transduction of T cells with lentiviruses

The activated cultured T cells were taken out, and polybrene at a final concentration of 8 µg/ml was added and mixed well. The lentiviral vector was slowly added at MOI=2. After well mixed, the mixture was placed in a centrifuge and centrifuged at 1500 rpm for 1.5 h. After that, it was placed in an incubator for culture for at least 24 h under the culture condition of 37°C +5% CO₂.

### 4. Expansion culture of transduced T cells

The transduced cells were taken out and the cell density was monitored to keep it at (0.5~1)×10⁶ cells/ml for use in subsequent examples.

The T cells obtained by transducing T cells with lentivirus containing BY-01G-BY-05G scFvs were named BCMA CAR-T cells BY-01G-BY-05G, respectively. Next, five CAR structures at the cellular level and animal level were selected to finally determine an optimal candidate scFv sequence.

### Example 2. Detection of CAR molecules expressed on the surface of BCMA CAR-T cells BY-01G-BY-05G

The CAR protein molecules expressed on the surface of five BCMA CAR-T cells obtained in Example 1 on the 5^{th} day after transduction were detected. The cells were stained with FITC-labeled BCMA antigen (ACRO Biosystems Cat. No. BCA-HF254), and were detected and analyzed by flow cytometry. The results were shown in Fig. 2. In addition to the very low CAR positive proportion (10.25%) and CAR expression level of BCMA CAR-T cell BY-03G (the abscissa value of the fluorescence intensity of CAR positive cells in Fig. 2 may refer to the CAR expression level), the CAR positive proportion and CAR expression level of BCMA CAR-T cells BY-01G, BY-02G, BY-04G, and BY-05G were relatively high, especially, the CAR positive proportion of BCMA CAR-T cell BY-02G reached 96.6%.

In addition, since BCMA antigen was used for staining in the experiment, this result also suggested that the ability of these CAR proteins to specifically recognize BCMA antigen was different. In subsequent experiments, various functional tests on the BCMA CAR-T cells BY-01G, BY-02G, BY-04G, and BY-05G that express better were performed, but further research on BY-03G that expresses worst was not performed.

### Example 3. Sustained proliferation of BCMA CAR-T cells

Antigen stimulation can activate CAR-T cells and cause CAR-T cell proliferation, while sustained activation of T cells will lead to cell exhaustion. The proliferation ability and effector function of exhausted T cells will slightly decrease, and the expression levels of some immune checkpoint genes will up-regulate. The persistence of CAR-T cells was verified by detecting the proliferation of BCMA CAR-T cells in multiple rounds of antigen stimulation experiments.

Before antigen stimulation, the CAR positive proportions of BCMA CAR-T cells in each group were adjusted to be consistent using untransduced T cells. In multiple rounds of antigen stimulation experiments, BCMA CAR-T cells in each group were co-cultured with BCMA positive target cell MM.1S (human multiple myeloma cells) in 24-well plate at an effector/target ratio of 1:1. 2ml of X-VIVO15 medium was used for each well, and 3 wells were repeated for each group of cells. 2 days later, 500 µl cells were taken out to perform CD3 and CAR staining with fluorescent-labeled CD3 antibody (BioLegend Cat. No. 317318) and BCMA antigen (same as Example 2). The cells were detected and analyzed by flow cytometry, showing the proportion, number, and fluorescence intensity of CAR positive cells in CD3 positive cells. The number of CAR positive cells in CD3 positive cells can also be calculated based on the conversion of volume multiples (CD3 is a marker that distinguishes whether they are T cells). Then each group was taken out based on the calculation results. CAR-T cells were added to MM.1S cells at an effector/target ratio of 1:1 for a new round of stimulation. This was repeated until the proliferation of CAR-T cells stagnated, and the antigen stimulation was terminated.

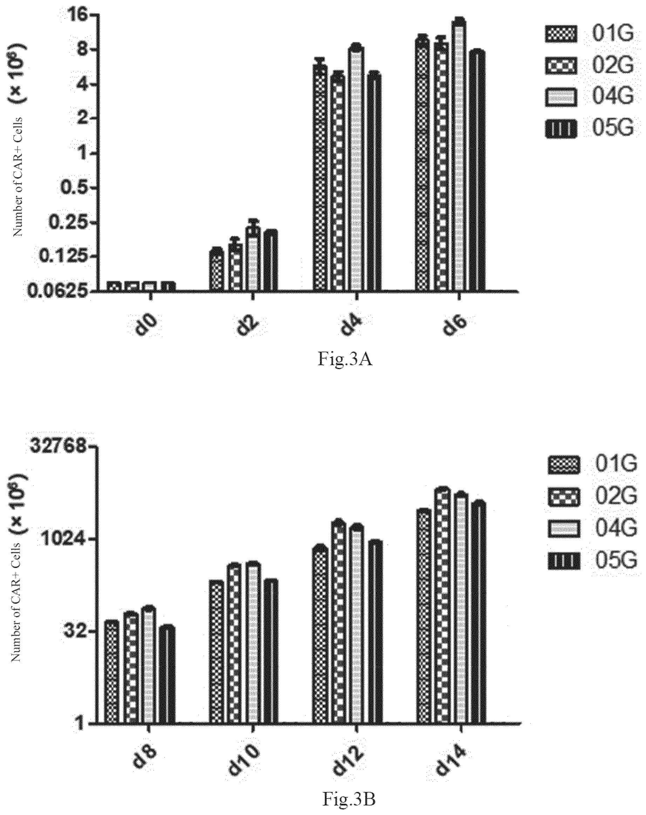

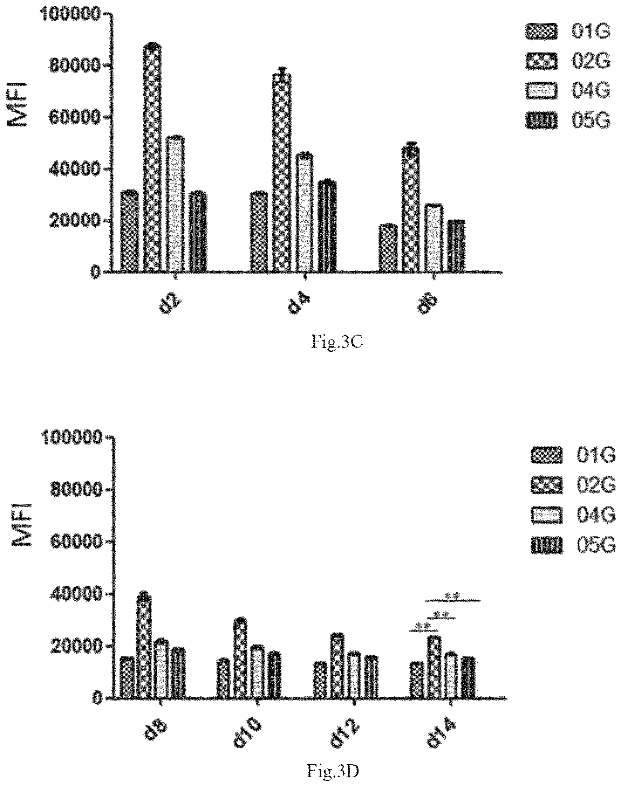

After multiple rounds of antigen stimulation, BCMA CAR-T cells in each group all showed obvious and massive proliferation (as shown in Fig. 3A and Fig. 3B). Flow cytometry was used to detect the CAR MFI value (average fluorescence intensity) of CAR positive cells in BCMA CAR-T cells after multiple rounds of antigen stimulation to reflect the average expression level of CAR on a single cell. The results were as follows: after 7 rounds of stimulation (Day 14), the CAR MFI value of the final BCMA CAR-T cell BY-02G group was the highest, with significantly higher than those of BY-01G, BY-04G, and BY-05G (the results were shown in Fig. 3C and Fig. 3D), indicating that after multiple rounds of antigen stimulation, the BCMA CAR-T cell BY-02G group had the highest average CAR expression level on a single cell.

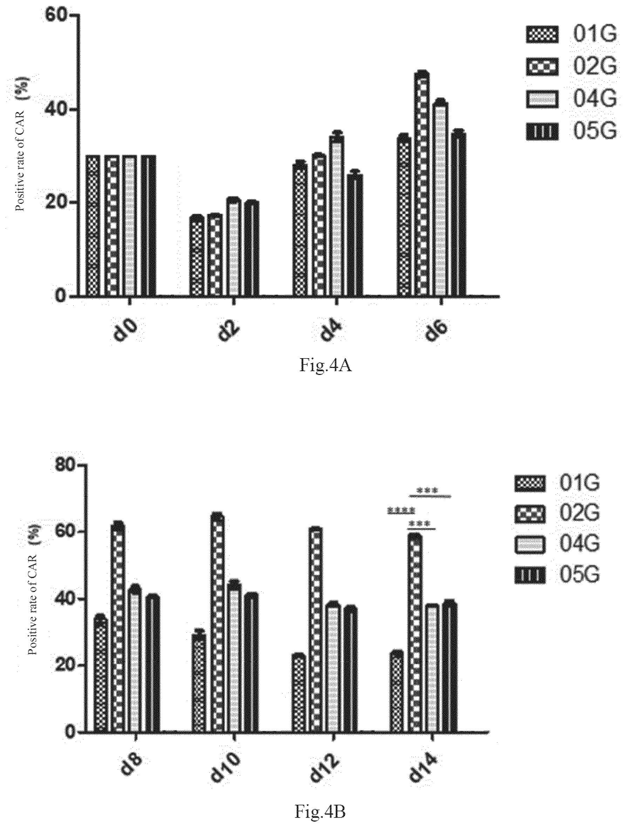

After multiple rounds of antigen stimulation, BCMA CAR-T cells in each group were able to continue to proliferate. Flow cytometry results showed that the BCMA CAR-T cell BY-02G group had the highest CAR positive proportion. When the total numbers of CAR⁺ cells were not significantly different, it indicates that its specific proliferation was the best, that is, the proliferation efficiency of CAR⁺T cells in the BY-02G group was better than that of non CAR⁺T cells. The specific results were as follows: after multiple rounds of antigen stimulation, the CAR positive proportion of BCMA CAR-T cells in each group changed significantly. From the 3rd round of stimulation (Day 6) to the 7th round of stimulation (Day 14), the CAR positive proportion of BCMA CAR-T cell BY-02G was significantly higher than those of BY-01G, BY-04G, and BY-05G. After 7 rounds of stimulation (Day 14), the final BCMA CAR-T cell BY-02G had the highest CAR positive proportion, with significantly higher than those of BY-01G, BY-04G and BY-05G (the results were shown in Fig. 4A and Fig. 4B).

### Example 4. Expression rates of immune checkpoint proteins PD1, Lag3, and Tim3 on the surface of BCMA CAR-T cells after sustained proliferation

According to the experimental method in Example 3, after completing multiple rounds of antigen stimulation (Day 14), the expression of immune checkpoint proteins on the surface of BCMA CAR-T cells was also detected. The immune checkpoint proteins include PD1, Lag3 and Tim3, and these proteins can also be used as markers of cell exhaustion. The expression levels of these immune checkpoint genes are up-regulated in exhausted T cells. The cells were stained with fluorescent-labeled PD1 antibody (BioLegend Cat. No. 329914), Lag3 antibody (BioLegend Cat. No. 369306), Tim3 antibody (BioLegend Cat. No. 345012) and BCMA antigen (same as Example 2), and were detected and analyzed by flow cytometry.

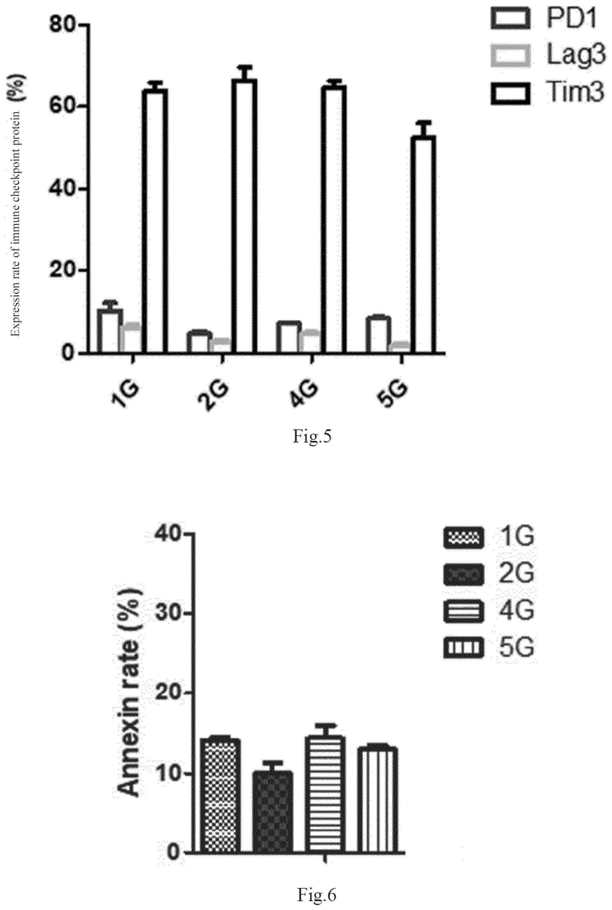

The results were shown in Fig. 5. The results showed the positive proportions of PD1, Lag3 and Tim3 in CAR positive cells in each group. BCMA CAR-T cell BY-02G had the lowest PD1 proportion and lower Lag3 proportion, which may indicate better cellular function.

### Example 5. Apoptosis level of BCMA CAR-T cells after sustained proliferation

Studies have shown that the proliferation of CAR-T cells after antigen stimulation is influenced by apoptosis. After completing antigen stimulation, the apoptosis levels of BCMA CAR-T cells in each group were detected. In normal cells, phosphatidylserine is only distributed on the inner side of the phospholipid bilayer of the cell membrane. In the early stages of cell apoptosis, phosphatidylserine will turn from the inner side to the outer side of the cell membrane. Annexin V, as a phospholipid-binding protein with high affinity to phosphatidylserine is used to detect apoptosis. The cells were stained with fluorescent-labeled Annexin V (BioLegend Cat. No. 640920) and BCMA antigen (same as above), and were detected and analyzed by flow cytometry.

According to the experimental method in Example 3, after completing multiple rounds of antigen stimulation (Day 14), the positive proportions of Annexin V of CAR positive cells in BCMA CAR-T cells in each group were also detected. The results were shown in Fig. 6. The positive proportion of Annexin V in BCMA CAR-T cell BY-02G was the lowest among BY-01G, BY-02G, BY-04G, and BY-05G, indicating a lower level of cell apoptosis.

### Example 6. Cytokine release experiments and cell killing experiments on BCMA CAR-T cells after sustained proliferation

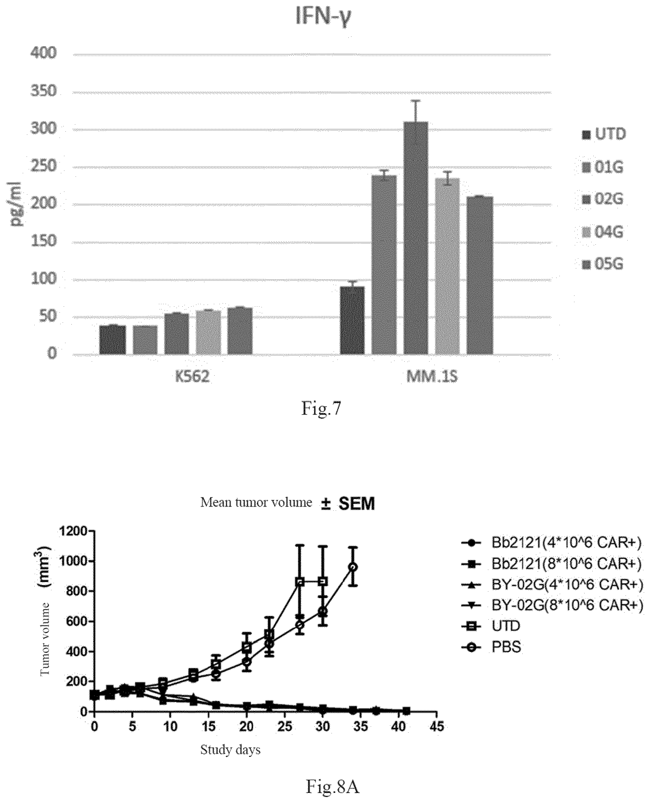

In order to further verify the persistence of BCMA CAR-T cell function, cytokine release experiments and cell killing experiments on CAR-T cells that have completed multiple rounds of antigen stimulation according to the experimental method in Example 3 were performed.
1) BCMA CAR-T cells were co-cultured with target cell in X-VIVO15 medium at an effector/target ratio of CAR positive cells to target cells of 1:1 for 24 hours. The target cell was MM.1S (this type of cell *per se* is a multiple myeloma cell line that endogenously expresses BCMA). K562 was a negative target cell control. The concentration of cytokine IFN-y in the cell supernatant was detected by ELISA. The results of the cytokine release experiment were shown in Fig. 7. The results showed that CAR-T cells in each group had the ability to release IFN-y, wherein BCMA CAR-T cell BY-02G released the highest amount of IFN-y after multiple rounds of antigen stimulation experiments, with significantly higher than those of other groups.
2) Each group of BCMA CAR-T cells were co-cultured with the above-mentioned target cell in X-VIVO15 medium at an effector/target ratio of 1:4 for 48 hours. The survival rate of target cell was detected by detecting the stable expression of luciferase activity in target cell. The results of cell killing experiments showed that after repeated antigen stimulation, all groups of BCMA CAR-T cells could kill 100% of BCMA positive target cell MM.1S. CAR-T cells had no obvious killing effect on the negative target cell K562.

Based on the above-mentioned experimental results, it can be seen that the developed BCMA CAR-T cells can maintain the ability to proliferate, recognize and kill tumor cells under the condition of repeated antigen stimulation, wherein the BCMA CAR-T cell BY-02G displayed better than those of other candidate CAR-T cells. It also indicated that CAR-T cells can have good persistence in the process of tumor treatment *in vivo.*

### Example 7. Anti-tumor activity of BCMA CAR-T cells in mice

Based on the *in vitro* pharmacodynamic test results, preliminary *in vivo* pharmacodynamic studies were also performed. Pharmacodynamic test was performed in mice. For *in vivo* experiments, two animal models (human multiple myeloma RPMI8226 cell in immunodeficient mice for subcutaneous tumor-bearing animal model, and human multiple myeloma MM.1S cell in immunodeficient mice for the tail vein tumor-bearing animal model) were selected. BCMA CAR-T cell BY-02G were further detected and BCMA CAR-T cell bb2121 from Bluebird Bio was used as a positive control (bb2121 scFv amino acid sequence is shown in SEQ ID NO: 40, and its nucleotide sequence is shown in SEQ ID NO: 41; except for the scFv sequences, the sequences of all other components in the construction of bb2121 CAR are identical to those of BY-02G), in order to verify whether BCMA CAR-T cell BY-02G has good anti-tumor function.

In the subcutaneous tumor-bearing animal experiment using RPMI8226 cell, two dose gradients for medium dose (4×10⁶ CAR⁺ cells/mouse) and high dose (8×10⁶ CAR⁺ cells/mouse) of CAR-T in the bb2121 group and BY-02G group were set. Each male NPI mouse was subcutaneously inoculated with 5×10⁶ RPMI8226 cells mixed with an equal volume of Matrigel for subcutaneous tumor-bearing. When the average subcutaneous tumor volume approached or reached 110mm³, a single tail vein injection of CAR-T cells was performed. Afterwards, the change in tumor volume was measured twice a week. The change curve of tumor volume was shown in Fig. 8A. The results of tumor volume change in mice showed that compared with non-transduced T cells, CAR-T in each experimental group had a good anti-tumor effect. The medium dose group had completely eliminated most of the subcutaneous tumors in mice after CAR-T injection for about 27 days, and both the medium dose and high dose groups performed equally in the bb2121 group and the BY-02G group.

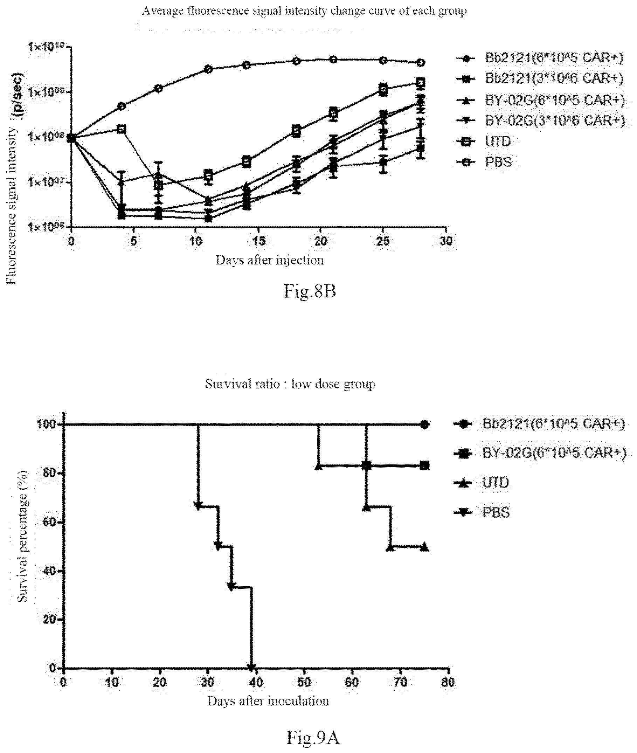

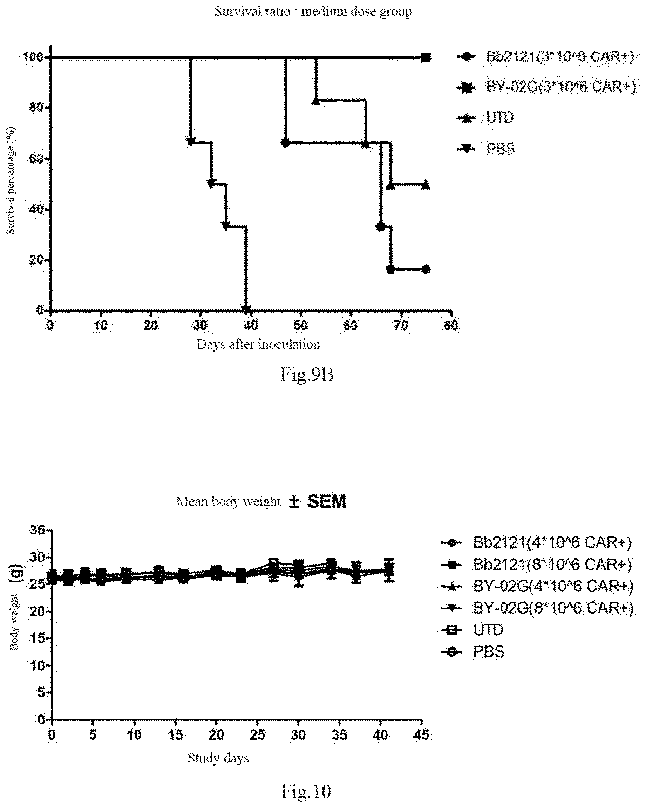

MM.1S-GFP-Luc cell (the method for preparing MM.1S-GFP-Luc cell: a lentiviral expression vector containing the green fluorescent protein GFP and the luciferase Luc coding region was constructed, then the lentivirus was packaged, MM.1S cell was transduced with lentivirus, positive monoclonal cells were sorted through flow cytometry using GFP signal, and the cells were identified through culture amplification and GFP expression; after the cell was determined as monoclonal, the cell preparation was completed) was inoculated into female B-NDG mice (body weight: 20 ± 2g) at 5×10⁶ cells/0.2ml per tail vein. On the 7th day after cell inoculation, the average fluorescence intensity of the group reached about 1×10⁸. According to the fluorescence signal intensity and body weight, the mice were randomly divided into groups and they were reinfused with the CAR-T cells. There were 6 mice in each group, totaling 6 groups, and thses 6 groups were as follows: G1: bb2121 (low dose 6×10⁵ CAR⁺ cells/mouse), G2: bb2121 (medium dose 3×10⁶ CAR⁺ cells/mouse), G3: BY-02G (low dose 6×10⁵ CAR⁺ cells/mouse), G4: BY-02G (medium dose 3×10⁶ CAR+ cells/mouse), G5: UTD, G6: PBS. After grouping, the tumor growth and body weight of the mice were observed, respectively, the tumor growth and body weight of the mice were measured and the measure values were recorded twice a week. 7 days after inoculation, when the average fluorescence intensity of the group reached about 1 × 10⁸, the mice were reinfused with CAR-T cells through a single tail vein injection. Afterwards, the changes in fluorescence signal intensity were measured twice a week. The change curve of fluorescence signal intensity until the 28th day was shown in Fig. 8B (D0 in Fig. 8B was calculated starting from the time of CAR-T single injection, i.e., 7 days after inoculation). The results showed that compared with untransferred T cells, CAR-T in all experimental groups had a good anti-tumor effect. At D28, BY-02G and bb2121 groups performed equally in the low dose group, while bb2121 group performed slightly better in the medium dose group. However, BY-02G group had better safety compared with the bb2121 group in terms of survival period in the medium dose group (as shown in Fig. 9B). In the survival period observation, the results showed that in the low dose group, the survival rates of the BY-02G group and the bb2121 group were comparable (as shown in Fig. 9A), but in the medium dose group, all mice in the BY-02G group survived until the end of the experiment, while the survival rate in the bb2121 group was below 20% (as shown in Fig. 9B).

### Example 8. Safety of BCMA CAR-T cells

In order to further determine the safety of the developed BCMA CAR-T cells, the body weight of mice after CAR-T cell injection was monitored. The RPMI8226 subcutaneous tumor-bearing animal model with a higher dose of CAR-T injection was taken as an example, and the results showed (Fig. 10) that compared with the non-transduced T cells or PBS group, there was no significant difference in the body weight of mice injected with BCMA CAR-T cells. Animals can tolerate doses greater than 4×10⁸ CAR⁺ cells/kg. The safety study results of BCMA CAR-T cells showed that in higher dose treatment, compared with the control group non-transduced T cells or PBS group, mice in each experimental CAR-T group did not observe abnormal weight loss, suggesting that the safety of this CAR-T cell at higher dose injection. In the above-mentioned study on the effectiveness of inhibiting tumor growth, at lower dose, the developed BCMA CAR-T had significantly tumor inhibitory effect. Meanwhile, CAR-T cells lasted longer, indicating that the developed BCMA CAR-T had good persistence *in vivo.*

### Example 9. Specificity of BCMA CAR-T cells

The scFv sequence of BCMA CAR-T cells is derived from the monoclonal antibody sequence with BCMA as the antigen. There is a need that BCMA CAR only specifically recognizes BCMA, but does not recognize other proteins, so as to ensure safety while ensuring effectiveness. The activity of CAR-T cells after co-culture with BCMA-negative target cells was detected to verify the specificity of BCMA CAR-T cells.

Firstly, different target cells (including cell lines Huh-7, SK-MEL-1, Ovcar3, 293T and Jurkat cells with negative BCMA expression, also including the MM.1S cell line with positive BCMA expression) were stained with fluorescent-labeled BCMA specific antibody (BioLegend Cat. No. 357506) and the same fluorescent-labeled homologous non-specific IgG (BioLegend Cat. No. 400322), and detected and analyzed by flow cytometry to detect the expression of BCMA protein on the surface of the target cells. Then, target cells with negative BCMA expression were co-cultured with T cells in X-VIVO15 medium at an effector/target ratio of 1:2. After 4 hours of co-culture, the expression of CD107a on the surface of CD8 positive T cells (i.e., CD3⁺/CD8⁺ T cells) was detected by flow cytometry. The expression of CD107a on the membrane was considered as a marker for activating cytotoxic lymphocytes (CD8⁺ T cells and NK cells). When cytotoxic lymphocytes were stimulated with specific antigens (i.e., immune activation was occurred) and expressed CD107a, CD107a can be used as the means to detect CAR-T cell specificity. The detection result of BCMA positive target cells co-cultured with T cells was used as a positive control.

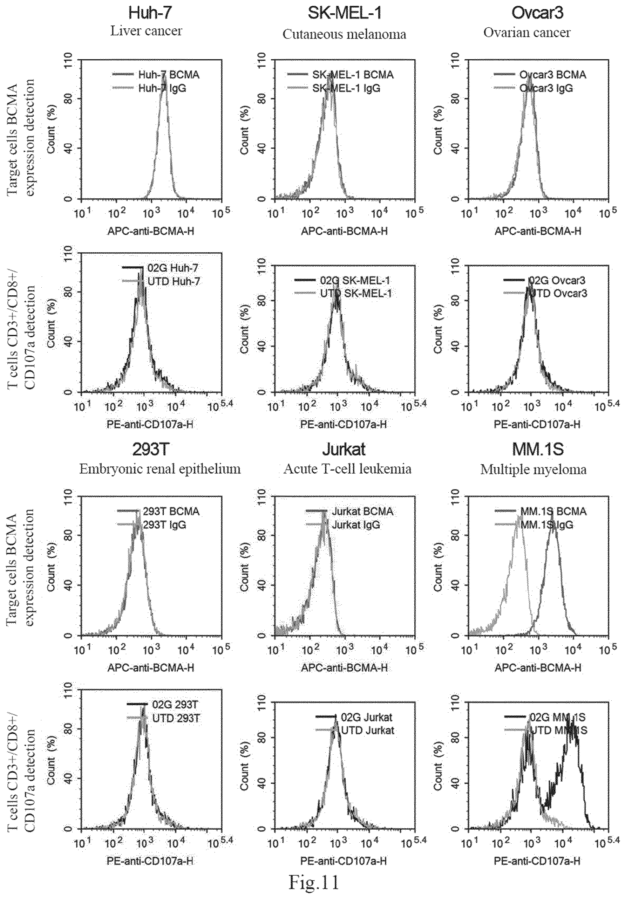

As shown in Fig. 11, in the detection result of BCMA protein expression, the staining results of various target cells with BCMA specific antibody were compared with the staining results of the same fluorescent-labeled homologous non-specific IgG (as shown in the 1st and the 3rd lines of Fig. 11), and only MM.1S cells, as a multiple myeloma cell line, had obvious BCMA protein expression (the double peaks of BCMA specific antibody staining and the same fluorescent-labeled homologous non-specific IgG staining were appeared in the figure of multiple myeloma in MM.1S). In the detection results of CD107a (as shown in the 2nd and the 4th lines in Fig. 11), after BCMA negative target cells were co-cultured with the untransduced UTD group T cells, the expression level of CD107a in CD8 positive T cells was almost identical, while after co-culturing MM.1S cells with BY-02G CAR-T cell, there was a significant proportion of CD107a positive T cells, indicating BY-02G CAR-T cell cannot recognize BCMA negative target cells and had good target specificity.

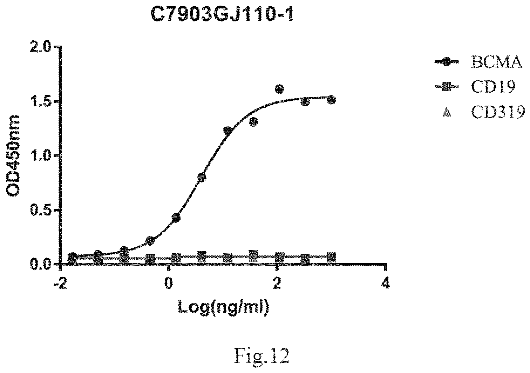

Furthermore, ELISA was used to further confirm the specificity of the scFv sequence of BY-02G (amino acid sequence as shown in SEQ ID NO: 11). Two proteins (CD19 and CD319) that shared the same B cells surface antigen as BCMA antigen were selected to perform well plate coating. Then, purified BY-02G scFv was used for affinity testing. The results were shown in Fig. 12. The scFv of BY-02G can only recognize and bind to BCMA antigen, demonstrating good antibody specificity.

In summary, *in vitro* pharmacodynamic test results showed that the expression of BY-02G CAR on the surface of T cells was superior to that of other candidate; and BCMA CAR-T cell BY-02G was superior to other candidate CAR-T in terms of CAR-T sustained proliferation, cell exhaustion level, apoptosis level, cytokine release, and *in vitro* cell killing. In BY-02G scFv, the amino acid sequences of the VH complementarity determining regions CDR1, CDR2, and CDR3 are the amino acid sequences as shown in SEQ ID NO: 1, SEQ ID NO: 2, and SEQ ID NO: 3, respectively, and the amino acid sequences of the VL complementarity determining regions CDR1, CDR2, and CDR3 of the scFv antibody are the amino acid sequences as shown in SEQ ID NO: 4, SEQ ID NO: 5, and SEQ ID NO: 6, respectively. In BY-02G scFv, the VH sequence of the scFv antibody is the amino acid sequence as shown in SEQ ID NO: 7, and the VL sequence of the scFv antibody is the amino acid sequence as shown in SEQ ID NO: 8.

*In vivo* animal experiments showed that BCMA CAR-T cell also had excellent performances in terms of specific recognition, tumor inhibition, and safety. The developed BCMA CAR-T can effectively recognize specific antigens and target cells, expressed and secreted large amounts of IFN-y and CD107a, thereby mediating corresponding immune responses. The developed BCMA CAR-T exhibited good safety in mice under high dose injection. The study results of *in vivo* anti-tumor activity showed that the developed BCMA CAR-T lasted for a long time *in vivo,* and can also achieve good tumor inhibitory effect at medium dose injection, indicating that the medium dose was safe and effective. The developed BCMA CAR-T also had good ability to recognize and kill tumor cells in animal tumor-bearing models *in vivo.* Moreover, under repeated antigen stimulation, the developed BCMA CAR-T cells can still maintain a high CAR positive rate, good cell proliferation ability and low apoptosis level, and can maintain effective killing on target cells.

### The Sequence Description

SEQ ID NO: 1: GFSLSTYH, which is BY-02G scFv VH CDR1;
SEQ ID NO: 2: ISSSGST, which is BY-02G scFv VH CDR2;
SEQ ID NO: 3: ARDLDYVIDL, which is BY-02G scFv VH CDR3;
SEQ ID NO: 4: PSVYNNY, which is BY-02G scFv VL CDR1;
SEQ ID NO: 5: ETS, which is BY-02G scFv VL CDR2;
SEQ ID NO: 6: AGTYVSGDRRA, which is BY-02G scFv VL CDR3;
SEQ ID NO: 7: Humanized BY-02G scFv VH amino acid sequence;
SEQ ID NO: 8: Humanized BY-02G scFv VL amino acid sequence;
SEQ ID NO: 9: Rabbit-derived BY-02G scFv VH amino acid sequence;
SEQ ID NO: 10: Rabbit-derived BY-02G scFv VL amino acid sequence;
SEQ ID NO: 11: Humanized BY-02G scFv amino acid sequence;
SEQ ID NO: 12: Rabbit-derived BY-02G scFv amino acid sequence;
SEQ ID NO: 13: Amino acid sequence of hinge region;
SEQ ID NO: 14: Amino acid sequence of transmembrane region;
SEQ ID NO: 15: Amino acid sequence of intracellular signal transduction region;
SEQ ID NO: 16: Amino acid sequence of costimulatory signal transduction region;
SEQ ID NO: 17: Amino acid sequence of the guiding peptide (i.e., signal peptide);
SEQ ID NO: 18: Nucleotide sequence encoding BY-02G scFv amino acid sequence;
SEQ ID NO: 19: BY-01G scFv amino acid sequence;
SEQ ID NO: 20: BY-03G scFv amino acid sequence;
SEQ ID NO: 21: BY-04G scFv amino acid sequence;
SEQ ID NO: 22: BY-05G scFv amino acid sequence;
SEQ ID NO: 23: Nucleotide sequence encoding BY-01G scFv amino acid sequence;
SEQ ID NO: 24: Nucleotide sequence encoding BY-03G scFv amino acid sequence;
SEQ ID NO: 25: Nucleotide sequence encoding BY-04G scFv amino acid sequence;
SEQ ID NO: 26: Nucleotide sequence encoding BY-05G scFv amino acid sequence;
SEQ ID NO: 27: BY-01G CAR amino acid sequence;
SEQ ID NO: 28: BY-02G CAR amino acid sequence;
SEQ ID NO: 29: BY-03G CAR amino acid sequence;
SEQ ID NO: 30: BY-04G CAR amino acid sequence;
SEQ ID NO: 31: BY-05G CAR amino acid sequence;
SEQ ID NO: 32: Nucleotide sequence encoding BY-01G CAR amino acid sequence;
SEQ ID NO: 33: Nucleotide sequence encoding BY-02G CAR amino acid sequence;
SEQ ID NO: 34: Nucleotide sequence encoding BY-03G CAR amino acid sequence;
SEQ ID NO: 35: Nucleotide sequence encoding BY-04G CAR amino acid sequence;
SEQ ID NO: 36: Nucleotide sequence encoding BY-05G CAR amino acid sequence;
SEQ ID NOs: 37-39: Amino acid sequence of the linker region linking VH and VL;
SEQ ID NO: 40: bb2121 scFv amino acid sequence;
SEQ ID NO: 41: Nucleotide sequence encoding the bb2121 scFv amino acid sequence.

## Claims

1. A BCMA-targeting chimeric antigen receptor, comprising an extracellular antigen recognition domain, a hinge region, a transmembrane region and an intracellular domain; wherein the extracellular antigen recognition domain comprises an anti-BCMA scFv antibody, the amino acid sequences of VH complementarity determining regions CDR1, CDR2, and CDR3 of the scFv antibody respectively comprise amino acid sequences as shown in SEQ ID NO: 1, SEQ ID NO: 2, and SEQ ID NO: 3; and the amino acid sequences of VL complementarity determining regions CDR1, CDR2, and CDR3 of the scFv antibody respecitvley comprise amino acid sequences as shown in SEQ ID NO: 4, SEQ ID NO: 5, and SEQ ID NO: 6.

2. The chimeric antigen receptor according to claim 1, wherein the scFv antibody is a humanized antibody; optionally, VH sequence of the scFv antibody comprises the amino acid sequence as shown in SEQ ID NO: 7, and VL sequence of the scFv antibody comprises the amino acid sequence as shown in SEQ ID NO: 8;
or, the scFv antibody is a rabbit-derived antibody;
optionally, VH sequence of the scFv antibody comprises the amino acid sequence as shown in SEQ ID NO: 9, and VL sequence of the scFv antibody comprises the amino acid sequence as shown in SEQ ID NO: 10.

3. The chimeric antigen receptor according to claim 2, wherein the scFv antibody has a linker region between VH and VL, and the linker region is selected from one or more of the following sequences: SEQ ID NOs: 37-39.

4. The chimeric antigen receptor according to claim 1, wherein the sequence of the scFv antibody is shown in SEQ ID NO: 11 or SEQ ID NO: 12.

5. The chimeric antigen receptor according to any one of claims 1-4, wherein the hinge region is derived from one or more of IgG1, IgG4, CD4, CD7, CD28, CD84, and CD8α; optionally, the amino acid sequence of the hinge region comprises amino acid sequence as shown in SEQ ID NO: 13;
and/or, the transmembrane region is derived from one or more of CD3, CD4, CD7, CD8α, CD28, CD80, CD86, CD88, 4-1BB, CD152, OX40, and Fc70; optionally, the amino acid sequence of the transmembrane region comprises amino acid sequence as shown in SEQ ID NO: 14;
and/or, the intracellular domain comprises an intracellular signal transduction region; optionally, the intracellular domain further comprises a costimulatory signal transduction region.

6. The chimeric antigen receptor according to claim 5, wherein the intracellular signal transduction region is derived from one or more of CD3ζ, CD3γ, CD3δ, CD3ε, CD5, CD22, CD79a, CD79b, FcRy, FcRβ, CD66d, DAP10, DAP12, and Syk; optionally, the intracellular signal transduction region is derived from CD3ζ; more optionally, the amino acid sequence of the intracellular signal transduction region comprises amino acid sequence as shown in SEQ ID NO: 15;
and/or, the costimulatory signal transduction region is derived from one or two or more of CD2, CD3, CD7, CD27, CD28, CD30, CD40, CD83, CD244, 4-1BB, OX40, LFA-1, ICOS, LIGHT, NKG2C, NKG2D, DAP10, B7-H3, and MyD88; optionally, the costimulatory signal transduction region is derived from CD28 or 4-1BB; more optionally, the amino acid sequence of the costimulatory signal transduction region comprises amino acid sequence as shown in SEQ ID NO: 16.

7. The chimeric antigen receptor according to any one of claims 1-4, wherein the chimeric antigen receptor further comprises a guiding peptide located at the N-terminus of the amino acid sequence of the chimeric antigen receptor; optionally, the guiding peptide is derived from CD8α; more optionally, the amino acid sequence of the guiding peptide comprises amino acid sequence as shown in SEQ ID NO: 17.

8. The chimeric antigen receptor according to claim 1 or 4, wherein the the chimeric antigen receptor comprises amino acid sequence as shown in SEQ ID NO: 28.

9. The chimeric antigen receptor according to any one of claims 1-4, wherein the extracellular antigen recognition domain further comprises a scFv antibody against one of the following targets: CD138, NKG2D, CD38, CS1, CD19, SLAMF7, CD70, CD44v6, and Lewis Y.

10. An isolated nucleic acid molecule comprising a nucleotide sequence encoding the chimeric antigen receptor according to any one of claims 1-9; optionally, the nucleic acid molecule comprises the nucleotide sequence as shown in SEQ ID NO: 18, or a nucleotide sequence having at least 70%, at least 75%, at least 80%, at least 85%, at least 90%, at least 95%, at least 96%, at least 97%, at least 98%, or at least 99% sequence identity to the nucleotide sequence as shown in SEQ ID NO: 18 and encoding the same chimeric antigen receptor; more optionally, the nucleic acid molecule comprises the nucleotide sequence as shown in SEQ ID NO: 33.

11. A vector comprising the nucleic acid molecule according to claim 10; optionally, the vector is an expression vector; more optionally, the vector is a viral vector; even more optionally, the vector is a lentiviral vector.

12. An engineered immune effector cell comprising the chimeric antigen receptor according to any one of claims 1-9, the isolated nucleic acid molecule according to claim 10, or the vector according to claim 11.

13. The immune effector cell according to cliam 12, wherein the immune effector cell is selected from one or more of T lymphocyte, natural killer cell, peripheral blood mononuclear cell, pluripotent stem cell, T cell differentiated from pluripotent stem cell, NK cell differentiated from pluripotent stem cell, and embryonic stem cell; optionally, the immune effector cell is T lymphocyte; more optionally, the source of the T lymphocyte is autologous T lymphocyte or allogeneic T lymphocyte.

14. A pharmaceutical composition comprising the engineered immune effector cell according to claim 12 or 13, and pharmaceutically acceptable adjuvant; optionally, the pharmaceutically acceptable adjuvant comprises a protective agent; more optionally, the protective agent comprises a cell cryopreservation solution.

15. The pharmaceutical composition according to claim 14, wherein the pharmaceutical composition is an intravenous injection.

16. Use of the chimeric antigen receptor according to any one of claims 1-9, the isolated nucleic acid molecule according to claim 10, the vector according to claim 11, or the engineered immune effector cell according to claim 12 or 13 in the preparation of a medicament for treating a disease or condition associated with the expression of BCMA.

17. The use according to claim 16, wherein the disease or condition associated with the expression of BCMA is cancer; optionally, the cancer is multiple myeloma; more optionally, the cancer is refractory or relapsed multiple myeloma.

18. The use according to claim 16, wherein the disease or condition associated with expression of BCMA is an autoimmune disease; optionally, the autoimmune disease is selected from the group consisting of systemic lupus erythematosus, rheumatoid arthritis, idiopathic thrombocytopenic purpura, myasthenia gravis, or autoimmune hemolytic anemia.

19. A method for treating a disease or condition associated with the expression of BCMA, including the following steps:
administering an effective amount of the engineered immune effector cell according to claim 12 or 13 or the pharmaceutical composition according to claim 14 or 15 to a subject having a need to treat a disease or condition associated with the expression of BCMA.

20. The method according to claim 19, wherein the disease or condition associated with the expression of BCMA is cancer; optionally, the cancer is multiple myeloma; more optionally, the cancer is refractory or relapsed multiple myeloma.

21. The method according to claim 19, wherein the disease or condition associated with expression of BCMA is an autoimmune disease; optionally, the autoimmune disease is selected from the group consisting of systemic lupus erythematosus, rheumatoid arthritis, idiopathic thrombocytopenic purpura, myasthenia gravis, or autoimmune hemolytic anemia.

22. The method according to claim 19, wherein the administration method is intravenous injection; optionally, the administration method is to administer an effective amount of the engineered immune effector cell according to claim 12 or 13 or the pharmaceutical composition according to claim 14 or 15 to a subject in a single injection; more optionally, the effective amount of the engineered immune effector cell or the pharmaceutical composition is at a dose of 1×10⁵ to 1×10⁷ cells/kg.

23. The engineered immune effector cell according to claim 12 or 13 or the pharmaceutical composition according to claim 14 or 15, for use in the treatment of a disease or condition associated with the expression of BCMA.

24. The engineered immune effector cell or the pharmaceutical composition for use in the treatment of a disease or condition associated with the expression of BCMA according to claim 23, wherein the disease or condition associated with the expression of BCMA is cancer; optionally, the cancer is multiple myeloma; more optionally, the cancer is refractory or relapsed multiple myeloma.

25. The engineered immune effector cell or the pharmaceutical composition for use in the treatment of a disease or condition associated with the expression of BCMA according to claim 23, wherein the disease or condition associated with expression of BCMA is an autoimmune disease; optionally, the autoimmune disease is selected from the group consisting of systemic lupus erythematosus, rheumatoid arthritis, idiopathic thrombocytopenic purpura, myasthenia gravis, or autoimmune hemolytic anemia.
